# EUROPEAN PATENT SPECIFICATION

(11) **EP 3 612 553 B1**
(45) Date of publication and mention of the grant of the patent: **08.06.2022**
(21) Application number: 18773868.7
(22) Date of filing: 19.04.2018
(51) Int. Cl.: C07K 14/34

(54) **AN IMPROVED METHOD FOR HIGH LEVEL PRODUCTION OF CRM**
VERBESSERTES VERFAHREN ZUR HOCHLEISTUNGSPRODUKTION VON CRM
PROCÉDÉ AMÉLIORÉ DE PRODUCTION À HAUT NIVEAU DE CRM

(30) Priority: 22.04.2017 IN 201741014335
(43) Date of publication of application: 26.02.2020
(73) Proprietor: Biological E Limited, Telangana, Hyderabad 500020 (IN)
(72) Inventor: MASILAMANI, Balamurali, Hyderabad Telangana 500020 (IN); SRIRAMAN, Rajan, Hyderabad Telangana 500020 (IN); DIXIT, Mandar Shirish, Hyderabad Telangana 500020 (IN); CHAKKA, Deviprasanna, Hyderabad Telangana 500020 (IN); SUREDDI, Satyam Naidu, Hyderabad Telangana 500020 (IN); MATUR, Ramesh Venkat, Hyderabad Telangana 500020 (IN); MANTENA, Narender Dev, Hyderabad Telangana 500020 (IN); DATLA, Mahima, Hyderabad Telangana 500020 (IN)
(74) Representative: Hoffmann Eitle
(86) International application number: PCT/IN2018/050235
(87) International publication number: WO 2018/193475

(56) References cited:
- EP-A2- 0 616 034
- WO-A1-2011/123139
- WO-A1-2013/068568
- WO-A1-2015/134402
- US-A1- 2004 087 020
- ZHOU J ET AL: "Secretory expression of recombinant diphtheria toxin mutants in B. Subtilis", JOURNAL OF TONGJI MEDICAL UNIVER, TONGJI MEDICAL UNIVERSITY, WUHAN, CN, vol. 19, no. 4, 1 January 1999 (1999-01-01), pages 253-256, XP009172483, ISSN: 0257-716X, DOI: 10.1007/BF02886955
- NAGARKAR P P ET AL: "The amino acid requirements of Corynebacterium diphtheriae PW 8 substrain CN 2000", JOURNAL OF APPLIED MICROBIOLOGY, WILEY-BLACKWELL PUBLISHING LTD, GB, vol. 92, no. 2, 1 January 2002 (2002-01-01), pages 215-220, XP002446474, ISSN: 1364-5072, DOI: 10.1046/J.1365-2672.2002.01521.X

## Description

### Field of the Invention

The present invention relates to an improved method for the production of CRM₁₉₇ with high yield using engineered *Corynebacterium diphtheriae* strain having increased copy number of CRM₁₉₇ gene.

### Background of the Invention

CRM₁₉₇ is a genetically detoxified form of diphtheria toxin. A single mutation at position 52, substituting glutamic acid for glycine, causes the loss of ADP-ribosyltransferase activity of the native toxin. The structural basis of CRM₁₉₇ for the lack of toxicity has been elucidated and is widely used as a carrier protein for conjugate vaccines. CRM₁₉₇, like diphtheria toxin, is a single polypeptide chain of 535 amino acids (58.4 KD) consisting of two subunits (linked by disulfide bridges).

CRM₁₉₇ is used as a carrier protein in a number of approved conjugate vaccines such as Haemophilus influenza type b conjugate marketed under the trade name Hibtiter TM, 13-valent pneumococcal polysaccharide conjugate marketed under the trade name PREVNAR 13^{®} and the like.

Ruth M. Drew et al., Bacteriol. 1951 Nov; 62 (5): 549-59; disclosed a chemically defined media suitable for the production of high titer diphtherial toxin and summarized the amino acid requirements of *Corynebacterium diphtheriae,* Toronto strain of Park-Williams no. 8. The media contains amino acids which effectively replace the animal derived component i.e. casein hydrolysate. The amino acids include glutamic acid, cystine, proline, tryptophan, leucine, valine, methionine, and glycine.

Rappuoli et al; Applied and Environmental Microbiology 1983 Vol. 46(3):560-564 have disclosed that the nontandem double lysogens were stable and capable of giving high yields of CRM₁₉₇, up to threefold higher than monolysogens.

R Fass. et al., Applied Microbiology and Biotechnology; April 1995, Volume 43(1): 83-88; disclosed a high-density growth approach to produce mutated diphtheria toxin from two strains of Corynebacterium diphtheriae: C7 (β) (tox-201, tox-9) and C7 (β)(tox-107). The procedure involves the use of a modified, non-deferrated growth medium that provided fast and high-density growth of the bacteria, and which, when associated with simultaneous depletion of glucose and iron, enhanced the toxin production. Oxygen-enriched air was supplied to enable the bacteria to grow to a cell density giving an absorbance of 70 at 600 nm (15-20 g/L dry weight). The maximum toxin concentration in the culture supernatant was 150 mg/l.

Parag P.Nagarkar et al., Journal of Applied Microbiology 2002, 92, 215-220; disclosed the amino acid utilization pattern during growth of Corynebacterium diphtheriae and showed that only four of the nine amino acids tested, namely cystine, histidine, aspartate and methionine, were critical for growth and toxin production by Corynebacterium diphtheriae.

European Patent No. 1 849 860 B1 disclosed the use of proteinaceous material of non animal origin, such as proteins from soy beans, cotton seeds, potatoes, etc., as a medium constituent for the cultivation of pathogenic bacteria.

US Patent No. 6,962,803 B2 disclosed a method of purifying diphtheria toxin by means of fermenting a microorganism strain capable of producing diphtheria toxin, said method comprising adding glucose to a growing culture whereby the addition of glucose maintains microorganism growth effective to support diphtheria toxin production. It further disclosed that in addition to a carbon source, there are other minimum nutritional requirements for growth which include trace metals, phosphate, a nitrogen source, generally casamino acids and yeast extract.

US Patent Application Publication No. 2011/0097359 A1 disclosed a media for culturing a strain of *Corynebacterium diphtheriae* to produce a level of diphtheria toxin or an analog thereof, wherein the medium is substantiality free of animal derived products and comprises: water; a carbohydrate source; a nitrogen source; and a number of free amino acids in an initial concentration wherein the initial concentration of each free amino acid is not limiting for the level of production of the diphtheria toxin or the analog thereof. Further discloses that the carbohydrate source is free of glucose.

WO 2006/100108 A1 disclosed a fermentation process comprising a step of growing a strain of *Corynebacterium diphtheriae* in a medium within the fermenter under conditions of agitation sufficient to maintain a homogenous culture and limited aeration such that pO2 within the culture falls to less than 4% for the majority of the fermentation step. Further disclosed that the pH within the fermenter is held between 7.0 and 7.8 by the degree of aeration without requiring addition of acid or base.

The CRM₁₉₇ process is generally sensitive to small changes in process components as well as process parameters. The CRM₁₉₇ production from *Corynebacterium diphtheriae* is exercised across the globe albeit with limited success for commercial realization. None of the above references disclosed method of producing CRM₁₉₇ with high yields such as >150 mg/l using engineered *Corynebacterium diphtheriae* strain. The inventors of the present invention have developed a metabolic flux model for high yield production of CRM₁₉₇ using engineered *Corynebacterium diphtheriae* strain.

### Objective of the Invention

It is the main objective of the present invention to provide an improved process for the high level production of CRM₁₉₇.

Yet another objective of the present invention is to provide an improved process for the high level production of CRM₁₉₇ which is cost effective and can be used for manufacturing conjugate vaccines.

### Summary of the Invention

The present invention provides an improved method for the production of CRM₁₉₇ with high yield using engineered *Corynebacterium diphtheriae* strain having increased copy number of CRM₁₉₇ gene, wherein the method comprises growing the strain in a fermentation medium comprising one or more amino acids and is free of animal derived components.

The present invention provides an improved method for the production of CRM₁₉₇ which method comprises culturing the engineered *Corynebacterium diphtheriae* strain having increased copy number of CRM₁₉₇ gene in a fermentation medium which is free of animal derived components and comprises more than 10 amino acids and supplementing the medium with nutrients.

The present invention also provides an improved method for the production of CRM₁₉₇ which method comprises culturing the engineered *Corynebacterium diphtheriae* strain having increased copy number of CRM₁₉₇ gene in a fermentation medium which is free of animal derived components and comprises more than 10 amino acids and supplementing the medium with nutrients based on metabolic flux model.

### Brief Description of the Drawings

Figure 1- Construction of a plasmid designated as pBE33
Figure 2- The metabolic flux balance model flow chart.

### Detailed Description of the Invention

The present invention provides an improved method for the production of CRM₁₉₇ with high yield using engineered *Corynebacterium diphtheriae* strain having increased copy number of CRM₁₉₇ gene, wherein the method comprises growing the strain in a fermentation medium comprising more than 10 amino acids and is free of animal derived components.

Engineered *Corynebacterium diphtheriae* strain (C7Ep) of the present invention refers to the *Corynebacterium diphtheriae* having episomally placed multiple copies of CRM₁₉₇ gene regulated by the same mechanism that of host CRM₁₉₇ gene and the said strain's genetic background is single lysogen.

The amino acids used in the present invention are selected from Alanine, Arginine, Aspartic acid, Cysteine, Glutamic acid, Glutamine, Glycine, Histidine, Isoleucine, Leucine, Lysine, Methionine, Phenyl alanine, Proline, Serine, Threonine, Tryptophan, Valine and its salts and each amino acid is used in an amount of about 0.05 to 2 g/l.

The effect of amino acids, vitamins and the process conditions for the production of CRM₁₉₇ has been studied. It has been found that certain amino acids have shown negative effect on the growth of bacteria. However, it is found that the production of CRM₁₉₇ is increased if more than 10 amino acids are used in an optimum concentration. The concentration of amino acids is optimized using Plackett Burman design of experiments.

Plackett Burman designs are experimental designs for investigating the dependence of some measured quantity on a number of independent variables (factors), in such a way as to minimize the variance of the estimates of these dependencies using a limited number of experiments. The outcome showed amino acid such as Aspartic acid, Glutamine, Glycine isoleucine leucine, valine and at a temperature of 35 to 36 °C have positive impact on the CRM₁₉₇ synthesis and amino acids such as Alanine, Isoleucine and at temperature of 35 to 36 °C have negative effect in the synthesis of CRM₁₉₇. Several levels of Design of experiments were carried out at different concentrations to achieve high level production of CRM₁₉₇ . The use of tyrosine and asparagine resulted in decrease in the overall yield of CRM₁₉₇ and hence these amino acids are not part of the invention.

In a preferred embodiment of the present invention, the fermentation medium contains combination of Phenyl alanine, Arginine and one or more other amino acids wherein the amount of Phenyl alanine and Arginine used is about less than about 1g/L.

In a more preferred embodiment of the present invention, the fermentation medium comprise combination of Phenyl alanine, Arginine and one or more other amino acids wherein amount of Phenyl alanine is about 0.25 to 0.75g/l and Arginine is about 0.1 to 0.5 g/l.

The present invention provides an improved method for the production of CRM₁₉₇ using engineered *Corynebacterium diphtheriae* strain having increased copy number of CRM₁₉₇ gene, wherein the method comprises growing the strain in a fermentation medium comprising more than 10 amino acids, supplementing the medium with vitamins in the range of about 0.05 to 20 mg per liter, wherein the medium is free of animal derived components.

In another embodiment of the present invention, amino acids, vitamins, trace elements and the like are added as nutrients to the fermentation medium during the cultivation.

Various nutrients used in the present invention as supplements include vitamins selected from Nicotinic acid, Thiamine, Pantothenic acid, Biotin, Riboflavin, FolicAcid; Pimelic acid; phosphate, a nitrogen source and trace metals and the like and each vitamin is used in an amount in the range from about 0.05 to 20 mg per liter.

The fermentation medium used in the present invention is a non-defferated and completely free from animal derived components. Further, the medium is also devoid of traditional meat based Loeffler media and casamino acid based defferated low iron YC media. The components of fermentation medium of the present invention comprises Yeast Extract, Veg.Peptone, Potassium-dihydrogen phosphate (KH₂PO₄), Tryptophan, Glucose, YC Trace salt solution.

In another embodiment of the present invention, the medium composition for the cultivation of engineered *Corynebacterium diphtheriae* strain having increased copy number of CRM₁₉₇ gene comprises base fermentation medium comprising Yeast Extract, Veg.Peptone, Potassium-dihydrogen phosphate (KH₂PO₄), Tryptophan, Glucose, YC Trace salt solution; one or more amino acids, vitamins, trace elements and the like.

Suitable trace metals include Potassium, Magnesium, Calcium, Chloride, Choline, Copper, Manganese, Sulfate, Zinc and the like.

The present invention provides an improved method for the production of CRM₁₉₇ using engineered *Corynebacterium diphtheriae* strain having increased copy number of CRM₁₉₇ gene, wherein the method comprises supplementing the fermentation medium with nutrients based on metabolic flux model.

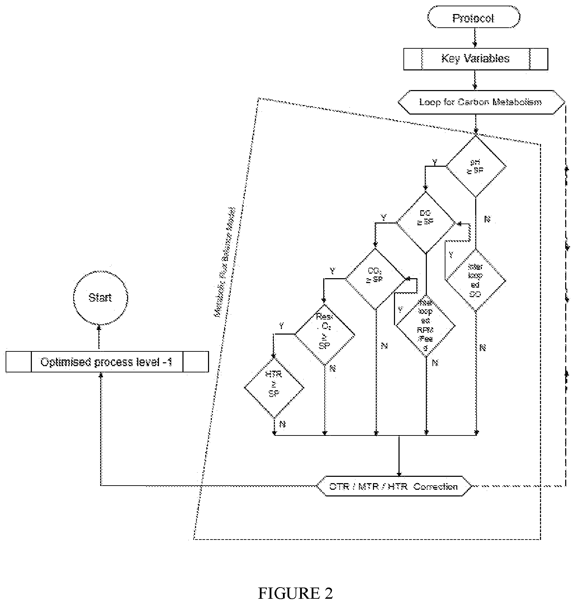

Metabolic flux model refers to the complete network of Heat Transfer, Mass Transfer, Oxygen transfer rate (OTR), Oxygen uptake rate (OUR) and Ion transfer kinetics wherein OTR is being maintained by means of agitation, back pressure and pump in pure oxygen. This model is depicted in Figure 2.

### Development of Metabolic flux model.

This model is created on the basis of online data interpretation of hydrogen ions accumulations in the process as a primary variable which is followed by the dissolved oxygen, oxygen to CO₂ conversion along with the heat liberated in the process. For example the model works on the carbon source limitation conditions. The carbon source is pulsed and the response is evaluated in the process. The corrective action was taken on hydrogen ions generation based on the response. Then the feed was optimized to get a steady pH and dissolved oxygen (DO) and heat transfer rate (For eg pH of 7.4; residual DO of > 2 to 20; Heat transfer rate HTR).

In an embodiment of the present invention, the method comprises supplementing the medium with glucose during the entire fermentation process.

The present invention does not involve the use of maltose as a carbon source and deferration step.

CRM₁₉₇ produced according to the present invention can be used for manufacturing conjugate vaccines such as pneumococcal conjugate, typhoid conjugate, Hib conjugate and the like.

In yet another embodiment, the present invention provides an improved method for the production of CRM₁₉₇ which method comprises culturing the engineered *Corynebacterium diphtheriae* strain having increased copy number of CRM₁₉₇ gene in a fermentation medium which is free of animal derived components and comprises more than 10 amino acids and supplementing the medium with nutrients based on metabolic flux model, wherein the amino acids are selected from Alanine, Arginine, Aspartic acid, Cysteine, Glutamic acid, Glutamine, Glycine, Histidine, Isoleucine, Leucine, Lysine, Methionine, Phenyl alanine, Proline, Serine, Threonine, Tryptophan, Valine and its salts.

In yet another embodiment, the present invention provides an improved method for the production of CRM₁₉₇ which method comprises culturing the engineered *Corynebacterium diphtheriae* strain having increased copy number of CRM₁₉₇ gene in a fermentation medium which is free of animal derived components and comprises more than 10 amino acids, wherein each amino acid is used in an amount of about 0.05 to 2 g/l.

In yet another embodiment, the present invention provides an improved method for the production of CRM₁₉₇ which method comprises culturing the engineered *Corynebacterium diphtheriae* strain having increased copy number of CRM₁₉₇ gene in a fermentation medium which is free of animal derived components and comprises more than 10 amino acids and supplementing the medium with nutrients based on metabolic flux model, wherein each amino acid is used in an amount of about 0.05 to 2 g/l.

In yet another embodiment, the present invention provides an improved method for the production of CRM₁₉₇ which method comprises culturing the engineered *Corynebacterium diphtheriae* strain having increased copy number of CRM₁₉₇ gene in a fermentation medium which is free of animal derived components and comprises base media, more than 10 amino acids and supplementing the medium with nutrients based on metabolic flux model, wherein each amino acid is used in an amount of about 0.05 to 2 g/l.

In yet another embodiment, the present invention provides an improved method for the production of CRM₁₉₇ which method comprises culturing the engineered *Corynebacterium diphtheriae* strain having increased copy number of CRM₁₉₇ gene in a fermentation medium which is free of animal derived components and comprises more than 10 amino acids and supplementing the medium with nutrients based on metabolic flux model, wherein the amino acids are selected from Alanine, Arginine, Aspartic acid, Cysteine, Glutamic acid, Glutamine, Glycine, Histidine, Isoleucine, Leucine, Lysine, Methionine, Phenyl alanine, Proline, Serine, Threonine, Tryptophan, Valine and its salts wherein each amino acid is used in an amount of about 0.05 to 2 g/l.

In yet another embodiment, the present invention provides an improved method for the production of CRM₁₉₇ which method comprises culturing the engineered *Corynebacterium diphtheriae* strain having increased copy number of CRM₁₉₇ gene in a fermentation medium which is free of animal derived components and comprises more than 10 amino acids, wherein the amino acids are selected from Alanine, Arginine, Aspartic acid, Cysteine, Glutamic acid, Glutamine, Glycine, Histidine, Isoleucine, Leucine, Lysine, Methionine, Phenyl alanine, Proline, Serine, Threonine, Tryptophan, Valine and its salts wherein each amino acid is used in an amount of about 0.05 to 2 g/l.

In a preferred embodiment, the present invention provides an improved method for the production of CRM₁₉₇ which method comprises
i) culturing the engineered *Corynebacterium diphtheriae* strain having increased copy number of CRM₁₉₇ gene in a fermentation medium which is free of animal derived components and comprises base media and more than 10 amino acids selected from Alanine, Arginine, Aspartic acid, Cysteine, Glutamic acid, Glutamine, Glycine, Histidine, Isoleucine, Leucine, Lysine, Methionine, Phenyl alanine, Proline, Serine, Threonine, Tryptophan, Valine and its salts and
ii) supplementing the medium with glucose and nutrients based on metabolic flux model.

In yet another embodiment, during the fermentation process temperature is maintained in the range from 30 to 40 °C and pH is maintained at 7.0 to 8.0, preferably 7.4 to 7.6 using 20% orthophosphoric acid, 12.5% ammonium hydroxide. The fermentation process is carried out for a period of 15 to 24 hours, preferably 16 to 20 hours.

In an embodiment, the present invention provides fermentation composition and process which is devoid of tyrosine and asparagine. In an embodiment, the process of the present invention is devoid of casamino acid based defferated low iron YC media, maltose as a carbon source and deferration step.

The present invention provides an improved method for the production of CRM₁₉₇ which method comprises culturing the engineered *Corynebacterium diphtheriae* strain having increased copy number of CRM₁₉₇ gene in a fermentation medium which is free of animal derived components and contains combination of Phenyl alanine, Arginine in an amount of about less than Ig/L and one or more other amino acids.

In an embodiment, the present invention provides an improved method for the production of CRM₁₉₇ which method comprises culturing the engineered *Corynebacterium diphtheriae* strain having increased copy number of CRM₁₉₇ gene in a fermentation medium which is free of animal derived components and comprises more than 10 amino acids and supplementing the medium with nutrients based on metabolic flux model, wherein the yield of CRM₁₉₇ obtained is more than 150 mg/L.

In an embodiment, the present invention enables making a conjugate vaccine which comprises conjugating polysaccharides from Salmonella typhi, Streptococcus pneumoniae, meningococcus, Haemophilus influenzae with CRM₁₉₇ prepared according to the present invention.

The present invention provides an improved method for the production of CRM₁₉₇ with high yield using engineered *Corynebacterium diphtheriae* strain having increased copy number of CRM₁₉₇ gene, wherein the method comprises growing the strain in media free of animal derived components comprising more than 10 amino acids and supplementing the medium with nutrients based on metabolic flux model.

In an embodiment, the present invention provides an improved method for the production of CRM₁₉₇ with yields such as 150mg/L, 200mg/L, 300mg/L, 500mg/L, Ig/L, 1.5g/L, 2 g/L, 2.5g/L, 3g/L, 3.5g/L, 4g/l, 4.5g/L, and 5g/L.

In an embodiment, the present invention provides an engineered *Corynebacterium diphtheriae* strain C7 (β 197) wherein pBE33 plasmid was transferred by electroporation.

The CRM₁₉₇ produced according to the present invention has been quantified with Immuno-Capture Enzyme Linked Immuno-Sorbent Assay (IC-ELISA).

Development of Engineered *Corynebacterium diphtheriae* strain having increased copy number of CRM₁₉₇ gene on an expression vector plasmid.

*Corynebacterium diphtheriae* C7 (β-197) ATCC 53821, the gene encoding for CRMi97 exists as single copy and mutant CRM₁₉₇ protein is secreted into the culture media under iron regulation. The yield of CRM₁₉₇ increases three-fold in *Corynebacterium diphtheriae* C7 strain that have two copies of the corynephage-beta (ATCC 39255). This suggests that gene copy number is linked to increased productivity. In order to make CRM₁₉₇ production from *Corynebacterium diphtheriae* commercially viable it is desired to increase the expression level. Integrating more copy number into bacterial genome is technically challenging. The multi copy phage integrants are genetically unstable and lose the additional copies of the CRM gene. The present inventors intended to improve the expression levels of the CRM₁₉₇ by increasing the gene copies of CRM₁₉₇ delivered on plasmids having an antibiotic selection marker, along with the native regulatory elements of CRM₁₉₇. Accordingly, the inventors have developed *Corynebacterium diphtheriae* strain having increased copy number of CRM₁₉₇ gene, evaluated the strain's stability. The modified strain had higher levels of CRM₁₉₇ expression compared to un modified *Corynebacterium* strains.

The present invention is more specifically illustrated with reference to the examples given below. However, it should be understood that the present invention is not limited by an example in any manner but includes variations thereof within the parameters described herein, as can be known to those well-versed in the art.

### Example 1

### Improvement of CRM₁₉₇ expression by episomal copy of CRM197:

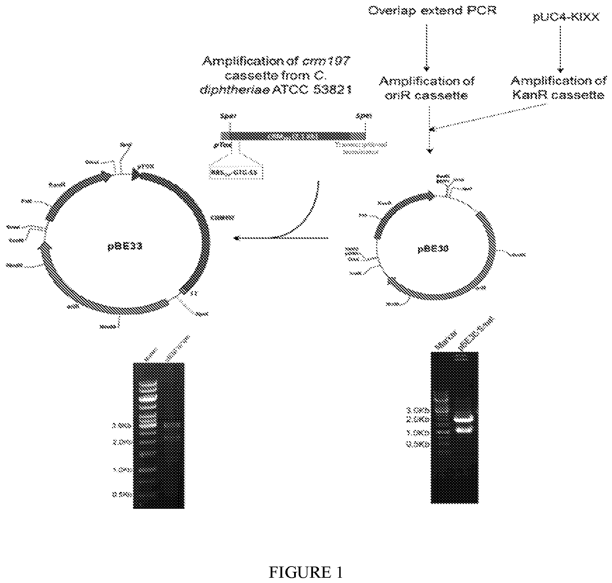

A vector that can stably replicate in *Corynebacterium diphtheriae* C7 (β 197) was prepared. Isolation of Native plasmid from *Corynebacterium* C7 *diphtheriae* has been carried out using large plasmid isolation protocol as described in T.C Currier et al., Anal Biochem. 1976; 76(2): 431441. Using the native plasmid DNA preparation, 1.87Kb origin of replication (oriR) was amplified. Simultaneously, 1.033Kb kanR sequence was amplified using pUC4-KIXX template DNA and blunt end ligated to oriR to generate pBE30. Further a 2.13 Kb gene sequence of CRMi97 comprising pTox promoter region (Boyd et al., 1988), cru and predicted terminator sequence was amplified from *Corynebacterium diphtheriae* C7 (β 197) genomic DNA and cloned into the unique Spel restriction site designed in the pBE30. The GAG mutation in this amplicon was verified by allele specific PCR assay (Pushnova et al., Analytical Biochemistry. 1998; 260: 24 to 29) and DNA sequencing. The plasmid thus obtained was designated as pBE33 (Figure 1).

The pBE33 plasmid was transferred by electroporation following the procedures optimized for *Corynebacterium diphtheriae* C7 (β 197). The transformed *Corynebacterium diphtheriae* C7 (β 197) (pBE33) colonies were confirmed by specific colony PCR and plasmid isolation and restriction digestion. Expression of CRM₁₉₇ in colonies was analyzed by SDS-PAGE and Western blotting. The CRM₁₉₇ in the media was quantified by ELISA and HPLC and were presented as concentration of CRM₁₉₇ expressed per liter of the culture media.

### Example 2

### Production of CRM₁₉₇ with engineered Corynebacterium diphtheriae C7Ep strain on base medium - free of animal derived components.

*Corynebacterium diphtheriae* C7Ep strain was used for this process. A two-step cultivation was followed for inoculum preparation in shake flask. Media for shake flask cultivation comprises of Yeast Extract (YE) 10 g/L, Veg. Peptone 15g/L, KH2PO4 4.3g/L, Tryptophan 50mg/L, Glucose 4.0g/L, Nicotinic acid 0.8 mg/L, Pimelic acid 0.08 mg/L, CuSO₄. 5H₂O 25 mg/L, ZnSO₄. 5H₂O 12.5 mg/L, MnCl₂. 4H₂O 6.25 mg/L, Cystine 0.5 g/L, Kanamycin 25 mg/L, Glucose 4.0 g/L.

To a 20 L fermenter medium, 5% inoculum was added to start the process. Medium for fermenter cultivation comprises of YE 15g/L, Veg.Peptone 30 g/L, KH₂PO₄ 4.3g/L, Tryptophan 50mg/L, Nicotinic acid 0.8 mg/L, Pimelic acid 0.08 mg/L, CuSO₄. 5H₂O 25 mg/L, ZnSO₄. 5H₂O 12.5 mg/L, MnCl₂. 4H₂O 6.25 mg/L, Cystine 0.5 g/L, Kanamycin 25mg/L, Glucose 4.0 g/L.

The culture was stirred at 300 RPM throughout the batch. Temperature was maintained at 35 °C and pH was maintained at 7.4 using 20% orthophosphoric acid and 5 N NaOH. The culture was aerated using 1.0 wm air. Culture was enriched with oxygen to maintain Dissolved Oxygen (DO) level to 20%. After first few hours, DO levels keeps falling below 20% when limit of O₂ enrichment is reached. The DO level for rest of the batch remains close to zero with rise towards end hours. When residual glucose in the broth falls below 0.5 g/L, 40% glucose feed was given with 2 g/L/hr. Batch was harvested after 14 hrs of cultivation when cell densities reached about 90 units of OD 600nm. The foam in the reactor was controlled by 30% organic antifoam. The CRM₁₉₇ production was reached to a titer of 60 to 65 mg/L of fermentation broth.

A fermentation run under similar conditions using unengineered *Corynebacterium diphtheriae* C7 CRM₁₉₇ strain produced about 35 to 40 mg/L of CRM₁₉₇ which is less than engineered strain. This shows the influence of the extra gene copies towards increased production of the CRM₁₉₇.

### Example 3:

Production of CRM₁₉₇ with engineered *Corynebacterium diphtheriae* C7Ep strain on base media free of animal derived components by following the metabolic flux balance model. *Corynebacterium diphtheriae* C7Ep strain was used for this process. A two-step cultivation was followed for inoculum preparation in shake flask. Media for shake flask cultivation comprises of YE 10 g/L, Veg. Peptone 15g/L, KH₂PO₄ 4.3g/L, Tryptophan 50mg/L, Glucose 4.0g/L Nicotinic acid 0.8 mg/L, Pimelic acid 0.08 mg/L, CuSO₄. 5H₂O 25 mg/L, ZnSO₄. 5H₂O 12.5 mg/L, MnCl₂. 4H2O 6.25 mg/L, Cystine 0.5 g/L, Kanamycin 25mg/L, Glucose 4.0 g/L.

To a 20 L fermenter medium, 5% inoculum was added to start the process. Medium for fermenter cultivation comprises of YE 15 g/L, Veg. Peptone 30 g/L, KH₂PO₄ 4.3g/L, Tryptophan 50mg/L, Nicotinic acid 0.8 mg/L, Pimelic acid 0.08 mg/L, CuSO₄. 5H₂O 25 mg/L, ZnSO₄. 5H₂O 12.5 mg/L, MnCl₂. 4H₂O 6.25 mg/L, Cystine 0.5 g/L, Kanamycin 25mg/L, Glucose 4.0 g/L.

The culture was stirred at 300 RPM throughout the batch. Temperature was maintained at 35 °C and pH was maintained at 7.4 using 20% orthophosphoric acid and 12.5% ammonium hydroxide. The culture was aerated using 1.0 wm air. Culture was enriched with oxygen to maintain DO level to 20%. A model was introduced which follows the metabolic rate of conversion in the given point of time, this model takes the input from the online parameter of residual DO in correspondence with the changes with respect to pH, CO₂, heat generation. In log phase, DO levels keeps falling below 20% despite O₂ enrichment limit has reached. The DO level for rest of the batch remains close to zero with rise towards end hours. When residual glucose in the broth fall below 0.5 g/L, 40% glucose feed was fed in order to justify the metabolic flux model. Batch was harvested after 14 hrs of cultivation when cell densities reached about 90 units of OD 600nm. The foam in the reactor was controlled by 30% organic antifoam. The CRM₁₉₇ production was reached to a titer of 150 mg/L of fermentation broth.

### Example 4:

### Production of CRM₁₉₇ according to the present invention

A three-step cultivation was followed for inoculum preparation. First two steps were done in the shake flasks. Medium for shake flask cultivation comprises of YE 10 g/L, Veg.Peptone 15g/L, KH₂PO₄ 4.3g/L, Tryptophan 50mg/L, Glucose 4.0g/L, YC Trace salt solution 2 ml/L, Cystine supplement 1 ml/L, Kanamycin 25mg/L, Glucose 4.0 g/L.

The base medium composition for seed fermenter was YE 15g/L, Veg.Peptone 30 g/L, KH₂PO₄ 4.3 g/L, Tryptophan 50mg/L, Nicotinic acid 0.8 mg/L, Pimelic acid 0.08 mg/L, CuSO₄. 5H₂O 25 mg/L, ZnSO₄. 5H₂O 12.5 mg/L, MnCl₂. 4H₂O 6.25 mg/L, Cystine 0.5 g/L, Kanamycin 25mg/L, Glucose 4.0 g/L

Given below is the fermenter medium composition (Table I) and the ingredients which has been designed following the Placket Burmann design.

**Table I**

| **S. No** | **Media components** | **Quantity** | **' Units** | **S. No** | **Media components** | **Quantity** | **Units** |
|---|---|---|---|---|---|---|---|
| 1 | Alanine | 0.1 | g/L | 18 | Valine | 1 | g/L |
| 2 | Arginine | 0.1 | g/L | 19 | Potassium | 2 | g/L |
| 3 | Aspartic acid | 0.5 | g/L | 20 | Magnesium | 1 | g/L |
| 4 | Cysteine | 0.5 | g/L | 21 | Thiamine | 0.1 | mg/L |
| 5 | Glutamic acid | 1 | g/L | 22 | Biotin | 4 | mg/L |
| 6 | Glutamine | 0.1 | g/L | 23 | Calcium | 2 | mg/L |
| 7 | Glycine | 0.5 | g/L | 24 | Chloride | 0.5 | mg/L |
| 8 | Histidine | 1 | g/L | 25 | Choline | 0.1 | mg/L |
| 9 | Isoleucine | 1 | g/L | 26 | Copper | 10 | mg/L |
| 10 | Leucine | 0.5 | g/L | 27 | Folic Acid | 1.6 | mg/L |
| 11 | Lysine | 0.1 | g/L | 28 | Manganese | 1 | mg/L |
| | | | | | | | |
| 12 | Methionine | 1 | g/L | 29 | Nicotinamic Acid | 100 | mg/L |
| 13 | Phenyl alanine | 0.5 | g/L | 30 | Pantothenic Acid | 50 | mg/L |
| 14 | Proline | 0.1 | g/L | 31 | Pimelic acid | 20 | mg/L |
| 15 | Serine | 0.1 | g/L | 32 | Riboflavin | 50 | mg/L |
| 16 | Threonine | 0.1 | g/L | 33 | Sulfate | 20 | mg/L |
| 17 | Tryptophan | 0.1 | g/L | 34 | Zinc | 7 | mg/L |

To a 20 L fermenter with above components with the base medium (YE 15 g/L, Veg.Peptone 30 g/L, KH₂PO₄ 4.3 g/L, Tryptophan 50 mg/L, Nicotinic acid 0.8 mg/L, Pimelic acid 0.08 mg/L, CuSO₄. 5H₂O 25 mg/L, ZnSO₄. 5H₂O 12.5 mg/L, MnCl₂. 4H₂O 6.25 mg/L, Cystine 0.5 g/L, Kanamycin 25mg/L, Glucose 4.0 g/L.), 5% inoculum from seed fermenter was added to start the process. Temperature was maintained at 35 °C and pH was maintained at 7.4 to 7.6 using 20% orthophosphoric acid, 12.5% ammonium hydroxide, and model based on metabolic flux (Figure 2) modulation of glucose feeding and OTR. The culture was aerated using 1.0 wm air. Culture was enriched with oxygen to maintain DO level to 20%. In log phase, DO levels keeps falling below 20% despite O₂ enrichment limit has reached. The DO level for rest of the batch remains close to zero with rise towards end hours. When residual glucose in the broth falls below 0.5 g/L, feed with 40% glucose solution was given at 4.5 g/L/hr. Intermittently vitamins and trace elements were supplemented at following levels; Nicotinic acid 6.425 mg/L, Pimelic acid 0.465 mg/L, CuSO₄. 5H₂O 25 mg/L, ZnSO₄. 5H₂O 12.5 mg/L, MnCl₂. 4H₂O 6.25 mg/L, Thiamine 0.08 mg/L, Pantothenic acid 0.25 mg/L, Biotin 0.006 mg/L, Riboflavin 0.3 mg/L, Folic Acid 0.06 mg/L . The batch was harvested after 18 hrs of cultivation when cell densities reached about 132 units of OD 600nm. The foam in the reactor was controlled by 30% organic antifoam. The CRM₁₉₇ production reached a titer of 450 to 500 mg/L of fermentation broth.

## Claims

1. An improved method for the production of CRM₁₉₇ with high yield using engineered *Corynebacterium diphtheriae* strain having increased copy number of CRM₁₉₇ gene, wherein the method comprises growing the strain in a non-defferated fermentation medium comprising more than 10 amino acids other than tyrosine and asparagine and being free of animal derived components, wherein no maltose is used as a carbon source.

2. The method as claimed in claim 1, wherein the method comprises supplementing the medium with nutrients.

3. The method as claimed in claim 2, wherein the supplement with nutrients is based on metabolic flux model.

4. The method as claimed in claim 1, wherein the amino acids are selected from Alanine, Arginine, Aspartic acid, Cysteine, Glutamic acid, Glutamine, Glycine, Histidine, Isoleucine, Leucine, Lysine, Methionine, Phenyl alanine, Proline, Serine, Threonine, Tryptophan, Valine and its salts.

5. The method as claimed in claim 4, wherein each amino acid is used in an amount of 0.05 to 2 g/l.

6. The method as claimed in claim 1, wherein the fermentation medium contains combination of Phenyl alanine, Arginine and other amino acids wherein amount of Phenyl alanine and Arginine is less than 1 g/L.

7. The method as claimed in claim 2, wherein the nutrients are selected from vitamins such as Nicotinic acid, Thiamine, Pantothenic acid, Biotin, Riboflavin, FolicAcid; pimelic acid; phosphate, a nitrogen source and trace metals.

8. The method as claimed in any of the preceding claims, wherein the fermentation medium is completely free from animal derived components.

9. The method as claimed in any of the preceding claims, wherein the fermentation medium comprises base fermentation media comprising Yeast Extract, Veg.Peptone, Potassium-dihydrogen phosphate (KH₂PO₄), Tryptophan, Glucose, YC Trace salt solution.

10. The method as claimed in any of the preceding claims, wherein the method comprises supplementing the medium with glucose during the entire fermentation process.

11. An improved method for the production of CRM₁₉₇ wherein the method comprises:
i) culturing the engineered *Corynebacterium diphtheriae* strain having increased copy number of CRM₁₉₇ gene in a non-defferated fermentation medium which is free of animal derived components and comprises base media and more than 10 amino acids selected from Alanine, Arginine, Aspartic acid, Cysteine, Glutamic acid, Glutamine, Glycine, Histidine, Isoleucine, Leucine, Lysine, Methionine, Phenyl alanine, Proline, Serine, Threonine, Tryptophan, Valine and its salts and
ii) supplementing the medium with glucose and nutrients based on metabolic flux model, wherein no maltose is used as a carbon source.

12. An improved method for the production of CRM₁₉₇, which method comprises culturing the engineered *Corynebacterium diphtheriae* strain having increased copy number of CRM₁₉₇ gene in a non-defferated fermentation medium which is free of animal derived components and comprises more than 10 amino acids other than tyrosine and asparagine and supplementing the medium with nutrients based on metabolic flux model, wherein each amino acid is used in an amount of 0.05 to 2 g/l, wherein no maltose is used as a carbon source.

13. The method as claimed in any of the preceding claims, wherein the fermentation is carried out at a temperature between 30 to 40 °C and at a pH in the range of 7.0 to 8.0. preferably 7.4 to 7.6

14. The method as claimed in any of the preceding claims, wherein the yield of CRM₁₉₇ obtained is more than 150 mg/L, 200 mg/L, 300 mg/L, 500 mg/L.

15. A method for manufacturing conjugate vaccine which comprises conjugating polysaccharides from *Salmonella typhi, Streptococcus pneumoniae, Haemophilus influenzae* with CRM₁₉₇ prepared according to any of the preceding claims.

## Patentansprüche

1. Verbessertes Verfahren zur Herstellung von CRM₁₉₇ mit hoher Ausbeute unter Verwendung von modifiziertem *Corynebacterium diphtheriae-Stamm* mit erhöhter Kopienzahl des CRM₁₉₇-Gens, wobei das Verfahren umfasst, dass man den Stamm in einem nicht-deferrierten Fermentationsmedium, das mehr als 10 Aminosäuren außer Tyrosin und Asparagin umfasst und frei von aus Tieren gewonnenen Komponenten ist, wachsen lässt, wobei keine Maltose als Kohlenstoffquelle verwendet wird.

2. Verfahren gemäß Anspruch 1, bei dem das Verfahren die Ergänzung des Mediums mit Nährstoffen umfasst.

3. Verfahren gemäß Anspruch 2, bei dem die Ergänzung mit Nährstoffen auf einem metabolischen Flussmodell beruht.

4. Verfahren gemäß Anspruch 1, bei dem die Aminosäuren ausgewählt sind aus Alanin, Arginin, Asparaginsäure, Cystein, Glutaminsäure, Glutamin, Glycin, Histidin, Isoleucin, Leucin, Lysin, Methionin, Phenylalanin, Prolin, Serin, Threonin, Tryptophan, Valin und ihren Salzen.

5. Verfahren gemäß Anspruch 4, bei dem jede Aminosäure in einer Menge von 0,05 bis 2 g/l verwendet wird.

6. Verfahren gemäß Anspruch 1, bei dem das Fermentationsmedium eine Kombination von Phenylalanin, Arginin und anderen Aminosäuren enthält, wobei die Menge von Phenylalanin und Arginin weniger als 1 g/l beträgt.

7. Verfahren gemäß Anspruch 2, bei dem die Nährstoffe ausgewählt sind aus Vitaminen, wie beispielsweise Nicotinsäure, Thiamin, Pantothensäure, Biotin, Riboflavin, Folsäure; Pimelinsäure; Phosphat, einer Stickstoffquelle und Spurenmetallen.

8. Verfahren gemäß mindestens einem der vorhergehenden Ansprüche, bei dem das Fermentationsmedium völlig frei von aus Tieren gewonnenen Komponenten ist.

9. Verfahren gemäß mindestens einem der vorhergehenden Ansprüche, bei dem das Fermentationsmedium Basisfermentationsmedien umfasst, die Hefeextrakt, veg. Pepton, Kaliumdihydrogenphosphat (KH₂PO₄), Tryptophan, Glukose, YC-Spurensalzlösung umfassen.

10. Verfahren gemäß mindestens einem der vorhergehenden Ansprüche, wobei das Verfahren die Ergänzung des Mediums mit Glukose während des gesamten Fermentationsprozesses einschließt.

11. Verbessertes Verfahren zur Herstellung von CRM₁₉₇, wobei das Verfahren die folgenden Schritte umfasst:
i) Kultivieren des modifizierten *Corynebacterium diphtheriae-Stamms* mit erhöhter Kopienzahl von CRM₁₉₇-Gen, in einem nicht-deferrierten Fermentationsmedium, das frei von aus Tieren gewonnenen Komponenten ist und Basismedien und mehr als 10 Aminosäuren umfasst, die ausgewählt sind aus Alanin, Arginin, Asparaginsäure, Cystein, Glutaminsäure, Glutamin, Glycin, Histidin, Isoleucin, Leucin, Lysin, Methionin, Phenylalanin, Prolin, Serin, Threonin, Tryptophan, Valin und ihren Salzen und
ii) Ergänzen des Mediums mit Glukose und Nährstoffen auf Basis eines metabolischen Flussmodells, wobei keine Maltose als Kohlenstoffquelle verwendet wird.

12. Verbessertes Verfahren zur Herstellung von CRM₁₉₇, wobei das Verfahren die Kultivierung des modifizierten *Corynebacterium diphtheriae-Stamms* mit erhöhter Kopienzahl von CRM₁₉₇-Gen, in einem nicht-deferrierten Fermentationsmedium, das frei von aus Tieren gewonnenen Komponenten ist und mehr als 10 Aminosäuren außer Tyrosin und Asparagin umfasst, und das Ergänzen des Mediums mit Nährstoffen auf Basis eines metabolischen Flussmodells, wobei jede Aminosäure in einer Menge von 0,05 bis 2 g/l verwendet wird und wobei keine Maltose als Kohlenstoffquelle verwendet wird, umfasst.

13. Verfahren gemäß mindestens einem der vorhergehenden Ansprüche, bei dem die Fermentation bei einer Temperatur zwischen 30 und 40°C und bei einem pH im Bereich von 7,0 bis 8,0, vorzugsweise 7,4 bis 7,6, durchgeführt wird.

14. Verfahren gemäß mindestens einem der vorhergehenden Ansprüche, bei dem die Ausbeute des erhaltenen CRM₁₉₇ mehr als 150 mg/l, 200 mg/l, 300 mg/l, 500 mg/l ist.

15. Verfahren zur Herstellung eines Konjugatimpfstoffs, das das Konjugieren von Polysacchariden aus *Salmonella typhi, Streptococcus pneumoniae, Haemophilus influenzae* mit CRM₁₉₇, das gemäß mindestens einem der vorhergehenden Ansprüche hergestellt wurde, umfasst.

## Revendications

1. Procédé amélioré de production de CRM₁₉₇ avec un rendement élevé en utilisant une souche modifiée de *Corynebacterium diphtheriae* présentant un nombre accru de copies du gène de CRM₁₉₇, dans lequel le procédé comprend la croissance de la souche dans un milieu de fermentation non déferré comprenant plus de 10 acides aminés autres que la tyrosine et l'asparagine et étant exempt de composants d'origine animale, dans lequel du maltose n'est pas utilisé comme source de carbone.

2. Procédé selon la revendication 1, dans lequel le procédé comprend la supplémentation du milieu avec des nutriments.

3. Procédé selon la revendication 2, dans lequel le supplément de nutriments est basé sur un modèle de flux métabolique.

4. Procédé selon la revendication 1, dans lequel les acides aminés sont sélectionnés parmi l'alanine, l'arginine, l'acide aspartique, la cystéine, l'acide glutamique, la glutamine, la glycine, l'histidine, l'isoleucine, la leucine, la lysine, la méthionine, la phénylalanine, la proline, la sérine, la thréonine, le tryptophane, la valine et ses sels.

5. Procédé selon la revendication 4, dans lequel chaque acide aminé est utilisé en une quantité de 0,05 à 2 g/l.

6. Procédé selon la revendication 1, dans lequel le milieu de fermentation contient une combinaison de phénylalanine, d'arginine et d'autres acides aminés dans lequel la quantité de phénylalanine et d'arginine est inférieure à 1 g/l.

7. Procédé selon la revendication 2, dans lequel les nutriments sont sélectionnés parmi les vitamines telles que l'acide nicotinique, la thiamine, l'acide pantothénique, la biotine, la riboflavine, l'acide folique; l'acide pimélique ; un phosphate, une source d'azote et les métaux à l'état de traces.

8. Procédé selon l'une quelconque des revendications précédentes, dans lequel le milieu de fermentation est complètement exempt de composants d'origine animale.

9. Procédé selon l'une quelconque des revendications précédentes, dans lequel le milieu de fermentation comprend un milieu de fermentation de base comprenant un extrait de levure, une peptone vég., du dihydrogénophosphate de potassium (KH₂PO₄), du tryptophane, du glucose, une solution saline YC Trace.

10. Procédé selon l'une quelconque des revendications précédentes, dans lequel le procédé comprend la supplémentation du milieu avec du glucose pendant tout le processus de fermentation.

11. Procédé amélioré de production de CRM₁₉₇ dans lequel le procédé comprend :
i) la mise en culture de la souche modifiée de *Corynebacterium diphtheriae* présentant un nombre accru de copies du gène de CRM₁₉₇ dans un milieu de fermentation non déferré qui est exempt de composants d'origine animale et comprend un milieu de base et plus de 10 acides aminés sélectionnés parmi l'alanine, l'arginine, l'acide aspartique, la cystéine, l'acide glutamique, la glutamine, la glycine, l'histidine, l'isoleucine, la leucine, la lysine, la méthionine, la phénylalanine, la proline, la sérine, la thréonine, le tryptophane, la valine et ses sels et
ii) la supplémentation du milieu avec du glucose et des nutriments sur la base d'un modèle de flux métabolique, dans lequel du maltose n'est pas utilisé comme source de carbone.

12. Procédé amélioré de production de CRM₁₉₇, lequel procédé comprend la mise en culture de la souche modifiée de *Corynebacterium diphtheriae* présentant un nombre accru de copies du gène de CRM₁₉₇ dans un milieu de fermentation non déferré qui est exempt de composants d'origine animale et comprend plus de 10 acides aminés autres que la tyrosine et l'asparagine et la supplémentation du milieu avec des nutriments sur la base d'un modèle de flux métabolique, dans lequel chaque acide aminé est utilisé en une quantité de 0,05 à 2 g/l, dans lequel du maltose n'est pas utilisé comme source de carbone.

13. Procédé selon l'une quelconque des revendications précédentes, dans lequel la fermentation est effectuée à une température entre 30 et 40 °C et à un pH dans la plage de 7,0 à 8,0, de préférence de 7,4 à 7.6

14. Procédé selon l'une quelconque des revendications précédentes, dans lequel le rendement en CRM₁₉₇ obtenu est supérieur à 150 mg/l, 200 mg/l, 300 mg/l, 500 mg/l.

15. Procédé de préparation d'un vaccin conjugué qui comprend la conjugaison de polysaccharides de *Salmonella typhi, Streptococcus pneumoniae, Haemophilus influenzae* avec une CRM₁₉₇préparée selon l'une quelconque des revendications précédentes.
